# EUROPEAN PATENT APPLICATION

(11) **EP 4 542 229 A2**
(43) Date of publication of application: **23.04.2025**
(21) Application number: 24207653.7
(22) Date of filing: 18.10.2024
(51) Int. Cl.: G01N 33/543, G01N 33/493, G01N 33/574, G01N 33/68, G01N 33/94

(54) **MARKER AND DEVICE FOR DIAGNOSING OVERALL HEALTH STATUS OF INDIVIDUAL**

(30) Priority: 20.10.2023 CN 202311368227
(71) Applicant: T&D Diagnostics Canada Pvt, Ltd, Cleveland, NS B0E 1J0 (CA)
(72) Inventor: Volk, Matthias, Cleveland, NS, B0E 1J0 (CA); Taylor, Peter, 55118 Mainz (DE)
(74) Representative: Petraz, Gilberto Luigi

(57) **Abstract**

The present invention provides a device for detecting an analyte in a liquid sample, including: a test element for testing presence or a quantity of the analyte in the liquid sample, where the analyte is one or more of analytes of neopterin; homocysteine; dopamine; or microalbuminuria.

## Description

### CROSS-REFERENCE TO RELATED APPLICATIONS

The patent application of the present invention claims a prior application in China, Application No.: 202311368227.7, filed on October 20, 2023, the specification, abstract, claims, and accompanying drawings of which are incorporated by reference in their entirety as a part of this application.

### BACKGROUND OF THE INVENTION

### Field of the Invention

The present invention relates to the field of health status prediction or diagnosis, and in particular to a method and a device which can quickly predict overall health status of an individual by testing a substance in a urine sample using rapid diagnosis technology.

### Description of the Related Art

The following description of the background art is merely a description of some background common knowledge, and does not limit the present invention in any way.

At present, detection devices or some methods for detecting whether a sample contains an analyte are widely used in hospitals or homes. These detection devices used for rapid diagnosis include one or more detection reagent strips, for example, for early pregnancy detection, drug abuse detection, etc. Such detection devices for rapid diagnosis are very convenient and can obtain detection results from the detection reagent strips in one minute or at most ten minutes or so. With the current popularity of home-based detection, many of these rapid diagnoses can be performed for testing at home.

For the diagnosis of specific diseases, it is also common to use the presence or absence of specific biomarkers in samples to diagnose or predict diseases, which is also used in hospitals or homes. These specific biomarkers, for example, for the detection of one or more analytes, are often associated with specific disease types. For example, in infectious disease testing, such as Coronavirus Disease 2019 (COVID-19) testing, it is to test whether there are antibodies or antigens associated with the novel coronavirus in the sample, so as to detect whether an individual is infected with the novel coronavirus. In acquired immune deficiency syndrome (AIDS) testing, for example, it is to test some substances associated with AIDS, such as human immunodeficiency virus (HIV) antibody testing, or the copy number of HIV nucleic acid, etc., to determine whether an individual is infected with HIV. For example, in a cancer test, certain specific markers are used to detect cancer-related markers in the sample of an individual, so as to determine whether the individual has cancer according to the content or level of the specific marker. Cancer markers are diverse, such as small molecule chemicals in blood samples, or autoimmune antibodies, or nucleic acid sequences based on nucleic acid genes, or the presence of proteins or protein mutations. In cancer detection, for example, as described in Chinese Patent Application No. 202110422182.1, a combination of some specific nucleic acid sequences is used to detect whether an individual has liver cancer; for example, as described in Chinese Patent Application No. 202310631059.X, a specific protein is used as a specific marker to diagnose whether an individual has lung cancer; and for example, as described in Chinese Patent Application No. 2023100823544, small molecule chemistry in blood or a combination of these compounds is used to diagnose whether an individual has lung cancer. In terms of cardiovascular disease detection, specific markers associated with cardiovascular disease are used to test whether an individual is at risk of a cardiovascular disease, such as troponin, myoglobin and natriuretic peptide, to test whether an individual is at risk of heart failure or myocardial infarction. In diabetes marker testing, the gold standard is to test the concentration of glucose in the blood to determine whether an individual has diabetes.

In addition, home-based self-detection is becoming increasingly common now, and most home-based self-detection products have appeared in COVID-19. Home-based detection of infectious diseases is becoming increasingly popular, which will also make it easier for individuals to test their own health at home, such as some self-testing or self-detection of certain specific diseases. However, existing traditional testing or detection, including home-based self-testing (over the counter, OTC), which diagnoses a certain specific disease, uses specific markers or analogs for detection in samples, and determines the diagnosis of a certain specific disease predicted by the latter according to the specific quantity or the degree of change in quantity.

This traditional testing is based on specific diseases for diagnosis. In fact, individuals only start testing when they develop a specific disease, such as cancer, an infectious disease, a cardiovascular disease, blood sugar, and other specific diseases, such as initial symptoms of a specific disease. Such testing appears to be relatively late in time, which requires earlier testing of physical health status of the individuals to provide early warning of the health status of the test individuals.

### BRIEF SUMMARY OF THE INVENTION

In order to overcome some defects of traditional technology, the present invention provides a set of markers that do not diagnose a specific disease, but rather provide an early overall evaluation of health status of an individual, and the test results indicate the overall health status of the individual. This overall health test can determine whether there is a risk of suffering from a specific disease in advance, so that interference or treatment will be performed in advance to avoid the specific disease.

Accordingly, in a first aspect of the present invention, the present invention provides a marker for testing overall health of an individual, where the marker is selected from one or a combination of more than one of markers of: neopterin; homocysteine; dopamine; or microalbuminuria. If any one marker is positive, the overall health status of the individual is indicated to be not very good. If a plurality of markers, such as two or more, are positive, the overall health status of the individual is not good, and the individual may have a possibility of developing a certain specific disease. Further use of a specific marker is needed to test or confirm a specific disease, or intervention or treatment is performed in advance. The positive and negative results here mean that these markers are compared with a preset cut-off value, and generally, if they are greater than the preset cut-off value, they are positive, and if they are smaller than the preset cut-off value, they are negative.

In a second aspect of the present invention, the present invention also provides use of a biomarker for preparation of a reagent for diagnosing overall health status of an individual, where the biomarker is selected from one or a combination of more than one of markers of: neopterin; homocysteine; dopamine; or microalbuminuria. The novel use of these markers is utilized to detect the overall health status of the individual, rather than for diagnosis of a specific disease. If any one marker is negative, the overall health status of the individual is indicated to be good, for example, in healthy status. If a plurality of markers, for example, two or more, are negative, the overall health status of the individual is indicated to be good or very good, and the individual may not have a possibility of developing a certain specific disease, or a possibility of developing a certain specific disease is very low. At this time, further use of specific markers is not needed to test or confirm a specific disease, and good living habits are still maintained to maintain such good physical health. If one marker is positive, the overall health status of the individual is indicated to be not very good. If test results of a plurality of markers are positive, the health status of the individual is indicated to be very poor.Then the individual needs to pay attention to its own health status or make a diagnosis of a specific disease. A method for the diagnosis is using a specific marker for a specific disease for testing. It can be understood that the more positive markers of the above markers indicate a degree of deterioration of overall unhealthy health status, the more positive markers indicate the unhealthier condition, and on the contrary, the more negative markers indicate the healthier condition.

In a third aspect of the present invention, the present invention provides a device for detecting an analyte in a liquid sample, including: a test element configured to test presence or a quantity of the analyte in the liquid sample, where the analyte is one or more of analytes of: neopterin; homocysteine; dopamine; or microalbuminuria. In some embodiments, a test strip is a lateral flow test strip. In some embodiments, the test strip is capable of simultaneously testing four analytes in the sample, and the analytes include a combination of markers of: neopterin; homocysteine; dopamine; or microalbuminuria. In some embodiments, the test strip is capable of simultaneously testing four analytes in the sample, and the analytes consist of analytes of: neopterin; homocysteine; dopamine; or microalbuminuria. With such a device, the individual subject can perform self-testing at home to pay attention to its overall health status or overall health condition, or changes in overall health status. For example, with the increase of the individual's age, some bad changes will occur in its physical health. With this device, the individual can perform testing many times and pay attention to the changes of its overall health state at any time, such as bad changes or good changes, such as becoming increasingly unhealthier, or becoming increasingly healthier. In some embodiments, for example, it is recommended to test twice a year if it is an individual aged 60 years or older, and at least once a year if it is an individual aged 40 years or older. The place of testing can be at home, or where the individual think it is appropriate to conduct self-testing.

In some embodiments, the test strip includes a detection area and a labeling area, the labeling area includes a first receptor capable of specifically binding to the analyte or marker and capable of flowing with the liquid sample, and the detection area includes and is fixed with a second receptor that specifically binds to a first receptor and is incapable of flowing with the liquid. In some embodiments, the first receptor includes an antibody, an antibody fragment, a polypeptide, or a polypeptide fragment, where the antibody is a monoclonal antibody. The second receptor includes an analog of the analyte. In some specific embodiments, the analogous substance of the analyte includes a linking reagent linked to the analyte, and the linking reagent can be linked by any chemical bond. For example, the linking is selected from one or more of reagents of: keyhole limpet hemocyanin (KLH), bovine gamma globulin (BGG), bovine serum albumin (BSA), bovine thyroglobulin (BTG), hen egg lysozyme (HEL), ovalbumin (OVA), sperm whale myoglobin (SWM), tetanus toxin (TT), methylated bovine serum albumin (mBSA), and rabbit serum albumin (RSA). These linking reagents are linked to the analyte or marker to make the marker substance have immunogenicity, and can be injected into an animal to produce an antibody. This immunogenic marker can be referred to as an antigen, while the marker of the present invention itself does not have immunogenicity and cannot produce an antibody in a mammal.

In a fourth aspect of the present invention, a device for detecting an analyte in a liquid sample is provided, including: 4 test elements, with each test strip test element for testing presence of the analyte or a quantity of the analyte in the liquid sample, or for testing presence of a marker or a combination of a plurality of markers in the liquid sample that is capable of predicting or diagnosing overall health status of an individual, where the analyte is one of analytes of: neopterin; homocysteine; dopamine; or microalbuminuria. In some embodiments, each test strip is used to detect a marker. In some embodiments, the test strip is a lateral flow test strip. In some embodiments, the test strip is capable of simultaneously testing four analytes in the sample, and the analytes include a group of: neopterin; homocysteine; dopamine; or microalbuminuria. In some embodiments, the test strip is capable of simultaneously testing four analytes in the sample, and the analytes consist of analytes of: neopterin; homocysteine; dopamine; or microalbuminuria.

In some embodiments, each test strip includes a detection area and a labeling area, the labeling area includes a first receptor capable of specifically binding to the analyte and capable of flowing with the liquid sample, and the detection area includes and is fixed with a second receptor capable of specifically binding to a first receptor and incapable of flowing with the liquid. In some embodiments, the first receptor on each test strip is capable of specifically binding to one of the 4 analytes, or the second receptor includes an analog of the analyte.

According to a fifth aspect of the present invention, a device for detecting an analyte in a liquid sample is provided, including: first, second, third, and fourth test elements, where the 4 test elements are located in one detection device, while the 4 test strips are capable of simultaneously contacting the liquid sample, where each test strip is capable of detecting one of four analytes of: neopterin; homocysteine; dopamine; or microalbuminuria. In some embodiments, the first test strip is used to test neopterin, and the second test strip is used to test homocysteine; and the third test strip is used to test dopamine, while the fourth test strip is used to test microalbuminuria. In some embodiments, the liquid sample includes urine, saliva or blood.

According to a sixth aspect of the present invention, a method for testing an analyte in a fluid sample is provided, including: providing a fluid sample of an individual, and detecting a content or a quantity of a marker in the fluid sample, where the marker includes one or more of neopterin; homocysteine; dopamine; or microalbuminuria; when any marker exceeds a certain cut-off value, overall health of the individual can be determined to be poor or unhealthy; and when any marker is lower than a certain cut-off value, overall health status of the individual can be determined to be good or healthy.

In some embodiments, when contents or quantities of the four markers all exceed a certain cut-off value, the overall health of the individual can be determined to be very poor or quite unhealthy, and when contents or quantities of the four markers are all lower than a certain cut-off value, the overall health of the individual can be determined to be very good or very healthy. The cut-off value here is not the same for each marker. If one of the markers exceeds the preset cut-off value, the individual is indicated to be unhealthy in some aspects, and can also be indicated to be overall unhealthy. On the contrary, if one of the markers does not exceed the preset cut-off value, the individual is indicated to be overall healthy or unhealthy in some aspects. In some embodiments, these four markers are used in combination, which can assess the overall health status of the individual. If only one of the markers is used, only assessment of a certain aspect can be completed, but not of the overall profile. These substances have a normal range in healthy people. If these marker substances exceed the normal range, the overall health of the individual is indicated to be problematic, and further examination is needed. This examination uses other more accurate markers for specific disease examination or reminds the individual to pay attention to its health status. Therefore, if all the four markers exceed their respective preset cut-off values, the health status of the individual is indicated to be not very good, and especially the overall health status is indicated to be not good, suggesting that a specific disease may develop. Of course, this specific disease may not appear immediately, or it may only appear within a certain period of time.

In some embodiments, a line appears in a test area of any one test strip, indicating that a result of a certain marker is negative, and no line appears in test areas of all test strips, indicating that all markers are positive. In some embodiments, if a line appears on a test line, a result is indicated to be negative, if a line does not appear on a test line, a result is indicated to be positive, and if a line does not appear on a test result control area of any test strip or test result control areas of all test strips, a test result is indicated to be invalid.

In some embodiments, a test device is provided, including four test strips on the device, each test strip testing a marker substance that can test the overall health of the individual and not a specific disease. The test strips of the device are contacted with the liquid sample at the same time, and the overall health of the individual is determined according to results of the test strips. In some embodiments, the test device includes an upper card and a lower card, the lower card has a tank structure carrying the test strips, and each test strip is disposed in the tank, where a sample application area of the test strip is exposed for contacting the liquid sample. During testing, urine is collected with a container, then the test device is inserted into the urine, that is, the exposed sample application area is allowed to contact the urine, and after waiting for a period of time, the number of lines appearing in the test areas of the test strips is checked, so as to determine the test result. According to the results, the overall health of the individual is determined.

In some embodiments, the sample is a urine sample for testing a marker in the urine sample, and this marker includes one or a combination of four of the labeling substances for assessing, testing or determining the overall health of the individual.

In a seventh aspect of the present invention, a content of neopterin (NPT) in urine can be used to test whether the urine is adulterated. Generally, neopterin (NPT) has a normal concentration in urine. If the urine is adulterated, e,g, added with water to dilute the urine, so that a concentration of NPT is reduced or cannot be tested, the urine may be considered to be adulterated. Therefore, if the NPT in the urine is lower than a certain preset cut-off value or cannot be detected, the urine is indicated to be adulterated, and on the contrary, a urine cup is indicated to be unadulterated. At the same time, if the concentration is higher than the preset cut-off value or too high, the individual is indicated to have a systemic health problem, such as a risk of suffering from cancer or an infectious disease in a later stage.

In the last aspect of the present invention, the invention provides a method to detest or determiner if an divisional is overall health or not overall health comprising these steps: providing a liquid sample of the individual; testing a quantity or a concentration of an analyte in the liquid sample; anddetermining the overall health of the individual according to the concentration or quantity tested,wherein the analyte comprises a combination of one or more of markers of: neopterin; homocysteine; dopamine; or microalbuminuria.

In some embodiments, the method further comprises comparing the concentration and the quantity of the analyte tested with a preset cut-off value, and predicting the overall, comprehensive or systemic health of the individual according to a result of the comparison.

In some embodiments, wherein when a concentration of any one analyte exceeds the preset cut-off value, the individual is indicated to be overall unhealthy; or when a concentration of any analyte is lower than the preset cut-off value, the individual is indicated to be overall healthy.

In some embodiments, when concentrations of the 4 markers all exceed the preset cut-off value, the individual is indicated to be overall, systemically and comprehensively physically very unhealthy, and the individual is very likely to be at risk of suffering from a specific disease in the future.

In some embodiments, the specific disease comprises one or more of cancer, an infectious disease, a cardiovascular disease, and diabetes.

In some embodiments, a concentration of the neopterin (NPT) exceeds the preset cut-off value, the individual is indicated to be overall unhealthy, and the individual is likely to be at risk of suffering from cancer or an infectious disease in the future. In some embodiments,when a concentration of the homocysteine exceeds the preset cut-off value, the individual is indicated to be overall unhealthy, and the individual is likely to suffer from a cardiovascular disease in the future. In some embodiments, when a concentration of the microalbuminuria exceeds the preset cut-off value, the individual is indicated to be overall unhealthy, and the individual is likely to suffer from diabetes in the future.

In some embodiments, providing a test device, wherein the device comprises 4 lateral flow test strips, and each test strip is capable of testing one of the 4 marker substances, allowing the 4 test strips to simultaneously contact the liquid sample, and then reading whether a color line appears in a test area on the test strips to obtain a test result. In some embodiments, wherein if a test result of any one test strip is negative, the concentration of the analyte tested is indicated to be lower than the preset cut-off value, and the individual is indicated to be overall healthy; and if a test result of a test area of any one test strip is positive, the concentration of the analyte tested is indicated to be higher than the preset cut-off value, and the individual is indicated to be overall unhealthy. In some embodiments, if a test result of each test strip is negative, concentrations of the 4 analytes tested are indicated to be lower than the preset cut-off value, and the individual is indicated to be overall very healthy; and if a test result of a test area of each test strip is positive, concentrations of the analytes tested are indicated to be higher than the preset cut-off value, and the individual is indicated to be overall very unhealthy.

In some embodiments, the liquid sample comprises one of a urine, saliva or blood sample.

In some embodiments, wherein the individual is at no risk of suffering from a specific disease or at little risk of suffering from a specific disease. In some embodiments, the specific disease comprises one or more of cancer, an infectious disease, a cardiovascular disease, and diabetes

### Beneficial effects

With the device and method of the present invention, 4 markers can be detected at one time, and these marker substances can indicate early physical condition of the individual. This early physical condition does not specifically refer to appearance of a specific disease, but indicates that the body as a whole may be at risk of suffering from a disease. In addition, some markers can be used to test urine adulteration, such as NPT, and can be used to test whether urine is adulterated when the overall health status is tested.

### BRIEF DESCRIPTION OF THE DRAWINGS

FIG. 1 is a schematic diagram of a test device according to one specific embodiment of the present invention.
FIG. 2 is a schematic diagram of a test device according to one specific embodiment of the present invention.
FIG. 3 is a structural schematic diagram of a test strip according to one specific embodiment of the present invention.
FIG. 4 is a structural schematic diagram of a test device according to one specific embodiment of the present invention.
FIG. 5 is a schematic diagram showing a positive test result of a carrier containing a test element according to one specific embodiment of the present invention, where FIG. 5A is a test image showing all positive results, FIG. 5B is a test image showing negative results for NPT and DOP and positive results for HCY and mALB, and FIG. 5C is a test image showing a negative result for mALB and positive results for the other three indicators.
FIG. 6 is a schematic diagram showing negative results for 4 markers of a test card according to one specific embodiment of the present invention.

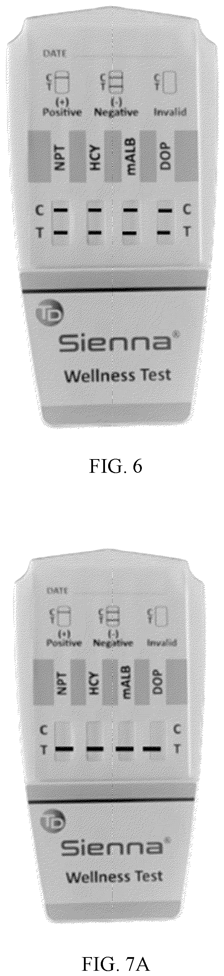

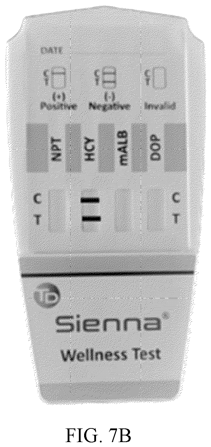

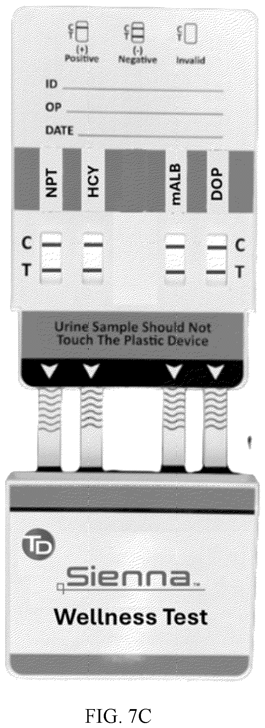

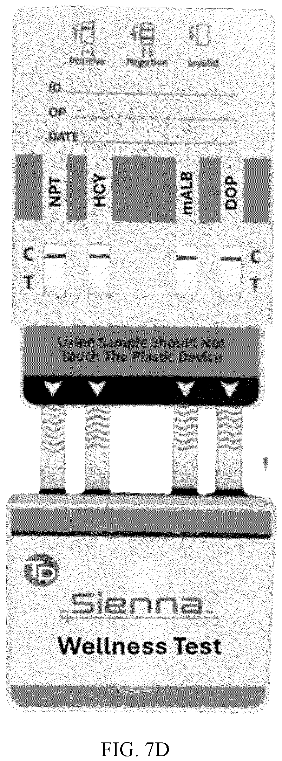

FIG. 7 is a schematic diagram showing an invalid test result of a test card according to one specific embodiment of the present invention, where FIG. 7A shows results in which all test results are invalid, FIG. 7B shows test results in which a negative result of HCY is valid and other test results are invalid, FIG. 7C shows test results in which all results are negative, indicating that the individual is in good health condition, and FIG. 7D shows a test in which all test results are positive, indicating that the individual is overall unhealthy and needs further detection or an appointment with a doctor.
FIG. 8 is a structural schematic diagram of a principle of testing a labeling substance according to one specific embodiment of the present invention.
FIG. 9 is a top view of a test strip according to one specific embodiment of the present invention.
FIG. 10 is a structural schematic diagram of a test strip according to one specific embodiment of the present invention.

### DETAILED DESCRIPTION OF THE INVENTION

The structures involved in the present invention or the technical terms used are further explained below. Unless otherwise specified, they shall be understood and explained according to the general terms commonly used in the art.

### Detection

Detection means to assay or detect presence or absence of a substance or a material, including but not limited to, a chemical substance, an organic compound, an inorganic compound, a metabolite, a drug, a drug metabolite, an organic tissue, a metabolite of an organic tissue, a nucleic acid, a protein, or a polymer. In addition, detection means that the quantity of a substance or a material is tested. Further, assay also means immunoassay, chemical assay, enzyme assay, and the like.

### Detection method

Any known method is used to test the markers of the present invention, and these markers are present in a liquid sample, such as urine. Test methods can be used, such as an isotope method: the method established by Refsum et al. in 1985, or chromatography: high-performance liquid chromatography (HPLC) is a relatively mature and widely used method. The shortcoming is that there are many variations in specimen treatment, chromatographic conditions, specimen detection, and quantification, which make it difficult to standardize. Full-automatic high-performance liquid chromatography is also a method. HPLC can be divided into a variety of methods according to the derivatization method (pre-column or post-column derivatization) and detection method (fluorescence, electrochemistry). HPLC is applied to accurately determine one of the four markers of the present invention, or the markers can be tested simultaneously. Test methods also include an immunological method: this method applies specific anti-4 marker technology, such as monoclonal antibodies, and uses a fluorescence polarization method or immunoassay to determine the 4 markers. The immunological method can adopt full-automatic chemiluminescence immunoassay technology to detect the 4 markers with instruments. This method is fast, simple to operate and highly automatic, can reduce human error, has good accuracy and precision, and is suitable for most clinical laboratory applications. Of course, chromatography technology can also be used, which is a disposable reagent strip that uses dry substances to be processed on a test strip (lateral flow) and is chromatographed in water. The disposable reagent strip is also referred to as a lateral flow test strip. Utilizing the test strip can quickly obtain test results within 3-10 minutes, and it is more suitable for non-professional testing, e.g. suitable for home-based self-testing, or belonging to OTC products, so that the results are obtained quickly and the operation is easy. Of course, any other appropriate test method can be used to test one of the 4 markers provided by the present invention or to test the 4 at the same time. For example, the 4 markers in a sample are tested simultaneously. For example, the presence or absence or content of the 4 markers in saliva, urine and blood are tested simultaneously, so as to test whether an individual is physically healthy according to the content of the 4 markers.

### Sample

The detection method or the detection device of the present invention can detect one or a combination of more than one of the above 4 markers in biological specimens. The biological specimens here include biological fluids (e.g. case fluids or clinical specimens). Liquid specimens or liquid samples, or fluid samples or fluid specimens may be derived from solid or semi-solid specimens, including excreta, biological tissues and food specimens. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological specimens" include animal, plant, and food-derived specimens, including, for example, human or animal-derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. The food specimens include food-processed materials, final products, meat, cheese, wine, milk, and drinking water. The plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other wastewater.

An appropriate detection element or test element according to the present invention can be used to detect any analyte. Preferably, the present invention is used to detect an analyte in saliva or urine.

### Downstream and upstream

Downstream or upstream is divided according to the flow direction of a liquid. Generally, a liquid flows from an upstream area to a downstream area. The downstream area receives a liquid from the upstream area, and a liquid also may flow to the downstream area along the upstream area. Here, downstream or upstream is generally divided according to the flow direction of a liquid, and for example, on some materials where capillary force is utilized to promote the flow of a liquid, a liquid may overcome gravity to flow towards an opposite direction to the gravity; and in this case, downstream or upstream is divided according to the flow direction of a liquid. For example, in the test device of the present invention, when a fluid sample or a liquid sample is absorbed by an absorbing element, a liquid can flow into a detection chamber from a liquid outlet of a capillary channel, contact with a test element 300 thereon, and flow into a sample application area 304 of the test element 300; in this case, the flow of the liquid in the sample application area 304 to an absorption area 303 is from upstream to downstream; and in a liquid flowing process, the liquid flows from a labeling area 307 to a testing area 306, and a detection area and a test result control area are provided on the testing area. The testing area may be a polyester fiber film, and the sample application area may be a glass fiber.

### Gas communication or liquid communication

Gas communication or liquid communication means that a liquid or a gas can flow from one place to another. In the flow process, the liquid or the gas may pass through some physical structures that play a guiding role. The "pass through some physical structures" here means that the liquid passes through the surface of these physical structures or the internal space of these structures and flows to another place passively or actively, where the passive flow is usually caused by external forces, such as the flow under the capillary action. The flow here may also be a flow due to self-action (gravity or pressure) of a liquid or a gas, and also may be a passive flow. The communication here does not mean that a liquid or a gas is necessarily present, but only in some cases indicates a connection relationship or state between two objects. If a liquid is present, it can flow from one object to another object. Here, it means a state in which two objects are connected. On the contrary, if there is no liquid communication or gas communication between two objects, and if a liquid exists in or on one object but cannot flow into or on another object, such a state is a non-communication, non-liquid communication or non-gas communication state.

### Test element

The "test element" here refers to an element that can be used to detect whether a sample or a specimen contains an analyte of interest. Such detection can be based on any technical principles, such as immunology, chemistry, electricity, optics, molecular science, nucleic acids, and physics. The test element can be a lateral flow test strip that can detect a variety of analytes. Of course, other appropriate test elements can also be used in the present invention,

Various test elements can be combined for use in the present invention. One form is a test strip. The test strip used to analyze an analyte (such as the 4 biomarker substances of the present invention or metabolites indicative of a physical condition) in a sample may be in various forms, such as in the form of an immunoassay or a chemical analysis. The test strip may adopt a non-competitive or competitive analysis mode. The test strip generally includes a water absorbent material having a sample application area, a reagent area and a testing area. A sample is added to the sample application area and flows to the reagent area through the action of capillary channels. In the reagent zone, if an analyte is present, the sample is bound to a reagent. The sample then continues to flow to the detection area. Other reagents, such as molecules that specifically bind to the analyte, are fixed in the detection area. These reagents react with the analyte (if any) in the sample and bind to the analyte in this area, or bind to a reagent in the reagent area. The label used to display a detection signal is present in the reagent area or a detached labeling area.

A typical non-competitive analysis mode is that if a sample contains an analyte or is higher than a preset cut-off value, a signal will be generated, indicating a positive result, and if a sample does not contain an analyte or is lower than a preset cut-off value, no signal will be generated, indicating a negative result. In a competitive method, if an analyte is not present in a sample or is less than a preset cut-off value, a signal is generated, indicating a negative result, and if an analyte is present or higher than a preset cut-off value, a signal is not generated, indicating a positive result.

The test element can be a test strip, which can be made of a water absorbent or non-water absorbent material. The test strip can include a variety of materials for liquid sample delivery. One material of the test strip can cover another material thereof. For example, filter paper covers a nitrocellulose membrane. One or more materials can be used in one area of the test strip, and one or more other different materials can be used in the other area. The test strip can be stuck to a certain support or on a hard surface for improving the strength of holding the test strip.

The analyte is detected through a signal-generating system. For example, one or more enzymes that specifically react with this analyte are used, and the above method of fixing a specific binding substance on the test strip is used for fixing a combination of one or more signal generating systems in the analyte detection area of the test strip. The substance that generates a signal can be in the sample application area, the reagent area or the testing area, or on the whole test strip, and one or more materials of the test strip can be filled with this substance. A signal-containing solution is added onto the surface of the test strip or one or more materials of the test strip are immersed in the signal-containing solution. The test strip containing a signifier solution added is allowed to be dried.

Various areas of the test strip can be arranged in the following way: a sample application area, a reagent area, a detection area, a control area, an area to determine whether a sample is adulterated or not, and a liquid sample absorption area. The control area is located behind the detection area. All areas can be arranged on one test strip that is only made of one material. Alternatively, different areas can be made of different materials. Each area can directly contact with a liquid sample, or different areas are arranged according to a flow direction of a liquid sample; and a tail end of each area is connected and superimposed with a front end of another area. Materials used can be those with good water absorption such as filter paper, glass fibers or nitrocellulose membranes. The test strip can also be in other forms.

Nitrocellulose membrane reagent strips are commonly used, that is, the testing area includes nitrocellulose membranes on which specific binding molecules are fixed to display a detection result; and other reagent strips such as cellulose acetate membrane or nylon membrane reagent strips may also be used. For example, reagent strips or devices containing reagent strips are described in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620; and US 6403383. The test strips disclosed in the above patent documents and similar devices with the test strips can be applied to the test element or detection device of the present invention for detection of an analyte, for example, detection of an analyte in a sample.

The detection reagent strips used in the present invention may be commonly referred to as lateral flow test strips, and the specific structures and detection principles of these detection reagent strips are known to those skilled in the art in the prior art. A common detection reagent strip includes a sample collection area or a sample application area 301, a labeling area 302, a detection area 303, and a water absorption area 304; the sample collection area includes a sample receiving pad, the labeling area includes a labeling pad, and the water absorption area may include a water absorbent pad, where the detection area includes necessary chemical substances for detecting the presence or absence of an analyte, such as immune reagents or enzyme chemical reagents. Generally, the commonly used detection reagent strip is the nitrocellulose membrane reagent strip, that is, the detection area includes the nitrocellulose membrane, and specific binding molecules are fixed on the nitrocellulose membrane to display the detection result; and other reagent strips such as cellulose acetate membrane or nylon membrane reagent strips can also be used. Of course, in the downstream of the detection area, there may also be a detection result control area. Generally, the control region and the detection region appear in the form of horizontal lines, a detection line (T line) or a control line (C line). Such a detection reagent strip is a conventional, and of course, other types of reagent strips for detection by capillary action may also be used. In addition, generally, the detection reagent strip has dry chemical reagent components, such as fixed antibodies or other reagents. When the reagent strip contacts a liquid, the liquid flows along the reagent strip with capillary action. With the flow, the dry reagent components are dissolved in the liquid, so as to go to the next area to treat the dry reagent in this area to react, so as to perform necessary detection. The liquid flow mainly relies on the capillary action. Here, all of the reagent strips can be applied to the detection device of the present invention or can be disposed in contact with liquid samples in a detection chamber or used for detecting the presence or absence of analytes in liquid samples that enter a detection chamber, or the quantity thereof. The test element is generally disposed in the test chamber 105, and when the test chamber contains a fluid sample, the fluid sample comes into contact with the test element and is assayed or detected.

In some embodiments, the device of the present invention includes 4 test strips, each test strip detecting an analyte, but these analytes are all detected in the same sample, such as urine. Therefore, during testing, all test elements are allowed to contact the liquid sample at the same time, and four indicators or 5 indicators can be detected at one time.

### Wellness or non-wellness

The analyte or marker substance of the present invention refers to those that can prompt, indicate, forebode, predict, detect, and determine the early health status of an individual in an early stage. The individual here refers to a human or a mammal. The health status herein includes the overall, comprehensive or general overall health status of an individual, but it is not possible to prompt, indicate, forebode, predict, detect, or determine a specific disease type of an individual. The overall health status here may be a overall health status level of 100%, may include an general or overall health status level of 80-95%, may include an overall health status level of 60-79%, or may include an overall health status level of 50% or more. The general or overall health status here also includes the concentrated manifestation of a variety of different factors affecting the physiology and psychology of individuals or the manifestation of overall results. Different factors herein include changes in the external environment, lifestyle habits of the individual, increasing age of the individual, and changes in one's own psychology, and one or a combination of more than one of these factors affect the health of the individual, such as physical and/or mental health.

As used herein, the term health status includes both wellness or non-wellness, and also refers to overall wellness or non-wellness. "Wellness" and "non-wellness" here are relative concepts, and wellness or non-wellness referred to in the present invention specifically refers to the overall, comprehensive or general health status of an individual, for example, being overall unhealthy, or being overall healthy, or being generally healthy, or being generally unhealthy.

Meanwhile, within the definition of the term "non-wellness" in the present invention, individuals who already have specific diseases are not included or excluded. This is a mere definition of technical terms, but does not exclude that individuals with specific diseases cannot be tested to assess general health with the device of the present invention. The main purpose is to test the level or condition of general health of individuals without specific diseases. That is, an early overall health assessment of individuals without specific diseases is performed by testing a liquid sample using one or a combination of the 4 specific markers screened according to the present invention, and then comparing the test results with a preset cut-off value to obtain overall health test results.

The meaning of such an individual already having a "specific disease" refers to an individual for whom the type of disease can be determined or specified by conventional means now, including a disease that can be confirmed by equipment or laboratory reagents, such as a specific disease that can be confirmed by computed tomography (CT), B-scan ultrasonography, X-rays, or other test reagents. The "specific disease" here refers to a certain symptom that has a clear name in medicine. For example, a general type of disease has broad classifications, and also more specific subclassifications under the broad classifications. Broad classifications include, for example, cancer or malignant tumors, infectious diseases, cardiovascular diseases, diabetes, or mental diseases, such as depression, schizophrenia, mania, etc. Subclassifications under broad classifications, such as cancer or malignant tumors, include all known cancer types such as lung cancer, intestinal cancer, pancreatic cancer, liver cancer, thyroid cancer, breast cancer, etc., as well as various stages or types under each particular cancer. For example, infectious diseases include HIV, novel coronaviruses, influenza viruses, and other infectious viruses. Cardiovascular diseases include heart diseases, such as acute or chronic myocardial infarction, or hemangioma, and the like. For example, diabetes includes type I or type II diabetes, and the like. All of the above belong to specific disease categories, and the specific marker substances screened by the present invention can evaluate, predict or test the overall health status of an individual before these specific diseases occur, it is predicted that a specific disease may occur or develop.

It is generally understood that an individual may already have unhealthy symptoms or unhealthy conditions, or may be overall unhealthy in the "early stage" of the occurrence of a specific disease, but there is no stage of the occurrence of specific disease symptoms. Thus, the marker of the present invention can be used to evaluate and test the overall health in an early stage, prompting the individual to pay attention to its physical condition, take necessary measures to improve his overall health, and avoid the occurrence of specific diseases, especially major diseases, such as malignant tumors or cancer, infectious diseases, cardiovascular diseases, psychological diseases, etc.; or further confirmation of specific diseases is needed, such as testing of cancer markers, such as tumor markers such as angiotensin converting enzyme (ACE), or markers of certain specific tumors such as lung cancer, intestinal cancer and other specific diseases for confirmation. If a specific disease is confirmed, intervention or treatment can be carried out early.

It can be understood that this overall unhealthy status does not mean that specific diseases will necessarily occur, and only means that the individual is not overall healthy and needs to take measures to pay attention to it. If no attention is paid to the status, a certain specific disease may occur, especially a major disease. This possibility is merely a hypothesis, and it does not mean that a specific disease will certainly occur. For the overall and comprehensive health test result obtained in this way, if the individual is the overall unhealthy, the individual is reminded to take some measures and pay attention to its own health. Of course, if the tested individual actually takes measures to make the individual from overall unhealthy to healthy, or overall and comprehensively healthy, the occurrence of specific diseases can be avoided, especially major diseases, in the later stage, or the occurrence of specific diseases can be delayed, life span can be prolonged, and the quality of life can be improved. If the test result is that the individual is overall healthy, the individual is indicated to be in good physical health, and the individual should continue to maintain is current condition.

The "early stage" here refers to the period before the individual does not have a specific disease. This period can be 1 year, 1-10 years, or earlier, including the time before the occurrence of a major disease. The specific disease here includes the above-mentioned definition of the specific disease. In one aspect, with the natural increase of an individual's age and gradual aging, the health status will change, for example, from healthy to unhealthy, and from overall health to overall unhealthy. At this time, no specific diseases appear. For example, if an average person is aged 35-40 years, a certain percentage of people will show overall unhealthy. If an average person is aged 40-60 years, a higher percentage of people will show overall unhealthy. In the present invention, through screening, it is found that some specific markers can evaluate, predict, test, detect, and confirm the early health status of individuals, especially the overall health status, and the early stage here can be divided by age. The above illustration of the definition of the early stage only uses increasing age as a factor, but it does not exclude those younger people, such as those under 35 years of age. In some developing countries, specific diseases increasingly occur in younger populations. Any one or a combination of the 4 markers screened according to the present invention can also be used to evaluate the early overall health of younger people to obtain overall health results, so as to prevent the occurrence of specific diseases, especially major diseases. For example, at the age of 15, 25, and 30 years, the reagents of the present invention can be used for testing.

Another aspect is the long-term influence of unfavorable factors in the external environment, which can also make individuals from overall healthy to unhealthy, thus eventually leading to the occurrence of specific diseases. These unfavorable factors in the external environment include factors that are not beneficial to individual health, such as people who receive ultraviolet rays and radiation for a long time, stay up late for a long time or people who should have bad habits. Such unfavorable factors of the external environment include those that are not beneficial to individual health, such as long-term exposure to ultraviolet rays and radiation, long-term staying up late or bad habits of people. These unfavorable factors will also lead to changes in the overall health status of individuals, which may eventually lead to the occurrence of specific diseases, especially the occurrence of major diseases. Such people can also be tested with the reagents of the present invention, such as markers, to performe the overall health test, to remind such people of their health status, and to take measures as soon as possible to avoid the occurrence of specific diseases, especially major diseases.

It can be understood that the occurrence and development of certain diseases is a long process, for example, of 5-10 years. Before the occurrence of a specific disease type, the health status of the body has already had problems, and at present, there is no good method to test and evaluate the overall health when these overall health problems appear. From this aspect, the present invention screens a group of specific markers, which can be used to test the overall health status of an individual in an early stage and predict the overall health status, so as to prevent the occurrence of a specific disease, especially the occurrence of major diseases. For infectious diseases, although the process from infection to symptom development is not long, for example, of 3-7 days, some people are not easily infected, and some people are easily infected. Most people who are easily infected are generally in poor overall physical condition, while those who are not easily infected are in better overall health. It can be understood that the occurrence of a specific disease in an individual is a manifestation of comprehensive factors, such as changes in immune ability, mental health, internal and external environmental factors, or other unfavorable factors acting on the individual, resulting in poor or very poor overall health status, thus leading to the occurrence of a specific disease. Therefore, from this perspective, it is more beneficial and significant to test the overall health or comprehensive health of individuals.

After long-term research and screening, the team of the present invention, i.e. the inventors of the present invention, have found that the following 4 markers can be used for combined detection or separate testing to predict the overall health risk, overall health status or general, systemic health status of the human body, and such prediction or testing is usually a prediction in the early morning. The "overall or general" here means that whether an individual has a catch-all or general, or comprehensive health risk can be predicted or tested, but a specific disease type cannot be indicated. In addition, this overall disease risk does not mean that a certain medically specific disease has been included or a certain medically specific disease is directly related, but indicates that the body has unhealthy condition or is overall healthy. In some embodiments, the four markers are: neopterin (NPT); homocysteine (HCY); dopamine (DOP); or microalbumin (mALB). These four markers can be used for testing together or separately, which will be elaborated and explained in detail below.

Generally, people have certain specific diseases or certain diseases, such as cancer or tumors, infectious diseases, blood sugar, or depression in the physiological sense, these only indicate a certain broad classification of diseases. If the content of any one of the above 4 markers in the human body is abnormal, that the body is indicated to have a health risk, and further testing is needed for clarification, such as testing using specific markers of tumors, infectious diseases, blood sugar, etc., such as testing by selecting markers directly associated with tumors or cancer, direct subjects of infectious diseases, such as antigens or antibodies, or direct markers of blood glucose diabetes, or other markers. The "further" here does not mean that there are immediate symptoms of a certain disease, but only means that there is a risk of developing a specific disease. The "specific" here is that it can be obviously directly associated with a certain disease. Using these direct markers, if the marker is at a certain level, it indicates a specific disease, such as cancer, a cardiovascular disease, an infection, or blood sugar. However, the above four markers of the present invention cannot be directly associated with a specific disease, but can be used to indicate that the body has a health risk, and indicate overall or systemic health status, especially in early testing before developing a specific disease.

### Neopterin and analogous substance thereof

In some embodiments, the inventors of the present invention have found that an abnormal elevation of neopterin ("NTP" for short) (chemical formula C9H11N5O4, CAS accession number 2009-64-5, molecular weight 253.2147, as shown in FIG. XX) in the human body indicates that the individual has an overall health risk, or is overall unhealthy, and is at increased risk of developing a particular disease, particularly a tumor or cancer, or/and an infectious disease, if not intervened early. Thus, it is suggested that the subject needs to further detect specific markers associated with tumors or antibodies or antigens associated with infectious diseases, or other specific markers associated with tumor cancer or infectious diseases. As explained earlier, if the level of NTP is higher than a normal level, it does not mean that the individual has a malignant tumor or cancer, or/and an infectious disease, but merely that there is a risk of developing a malignant tumor or cancer, or/and an infectious disease without intervention. If the result is positive and timely intervention is carried out to make the overall unhealthy status of the individual become overall healthy and NPT become negative, the risk of developing a malignant tumor or cancer, or/and an infectious disease is greatly reduced. Generally, there is a certain concentration of NPT in the urine of the human body. This concentration is generally stable or constant. If NPT is abnormally increased, it means that the human body is overall unhealthy and has an increased risk of suffering from a certain disease. This disease may be cancer or a tumor, or an infectious disease, such as an acute infection.

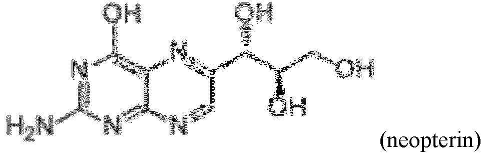

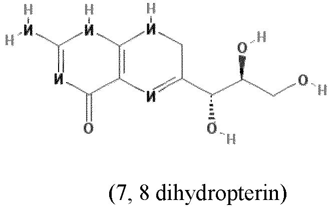

Neopterin and 7, 8 dihydropterin are the predominant pterins found in urine, of which neopterin accounts for 25-45% of total pterins. Neopterin and 7, 8 dihydropterin are the predominant pterins found in urine, of which neopterin accounts for 25-45% of total pterins (Wächter et al., 1992, Lindsay et al., 2014). 7, 8 dihydropterin is the main component of the rest of pterins found in urine. Both are produced by macrophages in immune system activation and inflammatory responses and are used as clinical markers of monocyte/macrophage activation. However, the present invention can employ both substances as markers of overall health. During testing, antibodies can be selected to recognize the main common functional groups of the two substances. Of course, antibodies that bind or recognize neopterin but do not recognize 7, 8 dihydropterin can also be selected. Therefore, 7, 8 dihydropterin is also a marker that can be used as a marker of the present invention to predict whether or not an individual is healthy and the health status of an individual.

In healthy adults, the mean urinary concentration of neopterin is approximately 125 µmol/mol creatinine. The urine concentration in urine is usually measured by creatinine to compensate for dilution of urine. The upper limit of normal neopterin (µmol)/creatinine (mol) in females is: 208 (18-25 years), 209 (26-35 years), 239 (36-45 years), 229 (46-55 years), 249 (56-65 years), 251 (over 65 years). The normal upper limit in males is slightly lower: 195 (18-25 years), 182 (26-35 years), 176 (36-45 years), 197 (46-55 years), 218 (56-65 years), 229 (over 65 years). These upper limits include 97.5% of healthy controls. The creatinine concentration varies widely depending on the subject's degree of hydration, but ranges around 12 mmol/l. A peak occurs late at night and early in the morning. Thus, the neoguanine concentration detected in the first morning urine was approximately 1500 nmol/L (379 ng/mL) (Wachter et al., 1989, Lindsay et al., 2014). The above concentration can be selected as a reference level or cut-off value for healthy people, and individuals above this cut-off value or reference level may have unhealthy performance. Of course, the higher the concentration or abnormally higher than the reference level, the overall health status is indicated to be poor or unhealthy, and in particular, treatment and intervention are needed, so as to reduce the risk of developing tumors or cancer, or/and an increased risk of infectious diseases.

### Neopterin and adulteration

In some embodiments, the NPT or 7, 8 dihydropterin of the present invention can also be used as a marker of urine adulteration. Traditional urine adulteration is to measure whether urine is adulterated by testing the specific gravity and PH value of urine. Urine adulteration is an abnormal behavior, but it does occur frequently in actual tests. For example, in recruitment, it is often necessary to test the candidate for drug abuse, but the candidate does not want to be tested positive, so the urine is adulterated by e.g. diluting the urine, e.g. adding a large amount of water to the urine detected for dilution to reduce the concentration of the analyte in the urine of the tested person to a level that cannot be tested. In addition, urine is simply not really collected and replaced with other solutions, the so-called artificial urine, which can also make the test results incorrect. In a person's urine, NPT or 7, 8 dihydropterin remains within a normal concentration range. If it is found that NPT is below the normal concentration or cannot be tested during testing, it means that the urine may be adulterated. This indicator can be used to test urine adulteration, which is much simpler than the traditional test of specific gravity and PH, and provides a new method to test urine adulteration. Of course, if the tested NPT concentration is abnormally elevated, it means that the urine is not adulterated, but it also means that the individual may be overall unhealthy, and the risk of cancer and acute infectious diseases in the later stage is increased. At that time, if it is necessary to eliminate the possibility of artificial injection or addition of NPT into urine, the suspicion or possibility of urine adulteration can be determined.

"Drug of Abuse" (DOA) here refers to the use of a drug (typically functions to paralyze nerves) not directed to a medical purpose. Abuse of these drugs will lead to physical and mental damage, dependency, addiction and/or death. Examples of abuse of drugs include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The detection device of the present invention may also be used to detect drugs belonging to a medical use but easy to be taken excessively, such as tricyclic antidepressants (imipramine or analogs) and acetaminophen. These drugs are metabolized into different micromolecular substances after being absorbed by human body. These micromolecular substances exist in blood, urine, saliva, sweat and other body fluids or in some body fluids.

### Homocysteine

In some embodiments, according to the present invention, for example, homocysteine ("HCY" for short) (chemical formula C4H9NO2S) is a marker associated with cardiovascular diseases. If the content of this substance in the human body is higher than a certain level, it means that the overall health is at risk, and the substance is also a marker for early prediction of cardiovascular diseases. Although it is known here that this marker is a marker associated with cardiovascular diseases, the present inventors have also found that this marker is also a marker associated with overall health, and if the marker is higher than a normal level, the test result is positive, which indicates that the overall and comprehensive health status of the individual is not good. Similarly, a positive result does not indicate that the individual necessarily already has a cardiovascular disease, but simply indicates that the individual is more likely to have a cardiovascular disease if no intervention is taken. If the HCY is kept below or equal to normal and the test result is negative, the individual is in good overall health condition and has a reduced risk of suffering from cardiovascular diseases. That is, markers have new functions and can be used to test, measure, predict, and diagnose the overall health status of an individual, i.e. whether it is good or bad, great or poor. As can be seen from the previous definitions of terms, the overall health status and specific diseases are different in definition, and the diagnostic significance and scope they represent are also different.

### Microalbumin

In some embodiments, for diabetes, glucose is not selected for testing, because glucose is a marker directly associated with diabetes, or hemoglobin A1c (HbA1c) is also a more common marker selected for diabetes, and microalbumin ("mALB" for short) is selected to predict diabetes, which is our brand-new discovery. Generally, in a healthy state, after renal filtration, microalbumin is in the blood, but if it is present in the urine, it is called urinary protein, which is also proven to be a marker of early diabetes, and the present invention discovers new markers for testing the overall health of an individual, by which the overall health of an individual can be tested, particularly by testing microalbumin in the urine. If the marker exceeds a normal level, it means that the overall health of the individual is not good, or is poor, and the individual is reminded of the need for attention and intervention. If no intervention is taken, the risk of suffering from specific diseases in the future will increase, and especially the risk of diabetes will increase. On the contrary, if intervention is taken to return the level of mALB to a normal healthy level, the overall health of the individual is restored to a healthy, good state, and the risk of suffering from a specific disease in the future is reduced, and for example, the risk of suffering from diabetes is reduced.

### Dopamine

Compared with physical diseases or health status, mental health has been paid more and more attention. The present inventors have found that for some mental health status, usually the following 4 substances are common markers of mental health, i.e. dopamine (DOP) (dopamine is a compound obtained by the hydroxylation reaction of phenylethylamine, the chemical name is 3,4-dihydroxyphenylethylamine. Its molecular formula is C8H11NO2, relative molecular mass is 153.18 g/mol), serotonin; cortisol; and epinephrine.

The present invention has found that adopting dopamine as a marker can display the early risk of human mental health, and compared with other substances, dopamine can show human mental health in an early stage, and is also used for testing the overall, general or comprehensive health status of an individual. Compared with several other substances, dopamine can better reflect the overall health status of individuals. For example, anxiety, increased sodium intake, mercury poisoning, primary aldosteronism, post-traumatic stress disorder, psychological stress, etc. will all increase dopamine levels. At the same time, compared with other substances, the high level of dopamine is also related to hyperactivity, inattention, emotional fluctuation, poor gastrointestinal function, psychosis, and sleep disorders. Therefore, selecting dopamine, a marker that can realize the evaluation of the overall health status of an individual, can test the overall health status of an individual early to avoid the occurrence of specific diseases in the future. Many studies have shown that long-term psychologically ill health can lead to physical harm, and long-term physical harm can lead to the occurrence of specific diseases, such as one or more specific diseases of depression, cancer, or cardiovascular diseases and diabetes.

Chronic abuse of illicit drugs such as methamphetamine increases dopamine above a certain level: the level of dopamine remains high even if a drug user is awake for a few days. Therefore, it's a sign of long-term abuse, for example, ice, similar to ethyl glucuronide (ETG) and alcohol abuse. Therefore, the dopamine test needs to rule out drug abuse. If methamphetamine is abused for a long time, the dopamine level will increase, but if drug abuse is ruled out, the dopamine level is still higher than a normal level, which means that the individual is overall unhealthy due to long-term stress. This unhealthy status may lead to specific diseases, which may be neurological or mental diseases, or may lead to other physiological diseases, such as cancer and infectious diseases.

### Four markers combined

The abnormality of the above four markers indicates that the content of these markers has increased relative to healthy people. The increase of the content has a standard or cut-off value. If it does not exceed a certain content, it means that the human body is very healthy. On the contrary, if it is higher than a certain set value, it means that the individual is not healthy or not very healthy overall. Of course, if all the 4 markers are elevated relative to a cut-off value at the same time, it indicates that the individual is very unhealthy overall, and the risk of suffering from specific diseases is high or the risk of containing specific diseases at the same time is increased. If only 3 markers are elevated relative to a normal cut-off level, it indicates that the overall health status of the individual is not very good, and the risk of suffering from specific diseases is relatively high; and of course, if only two or one of the markers are elevated relative to a normal cut-off level, it means that the individual has poor overall health and may be at high risk of suffering from specific diseases. On the contrary, if the levels of all 4 markers in an individual are at a healthy cut-off level, it means that the individual is overall physically healthy and at very low risk of suffering from specific diseases. According to different degrees, health status can be sorted as very healthy, healthy, generally healthy, unhealthy, quite unhealthy and very unhealthy. If all 4 markers are at a normal level, it means that the individual is very healthy, if all 4 markers are above a normal level, it means that the individual is quite unhealthy, if one or two of the 4 marker species are above a normal level, it means that the individual is generally healthy or unhealthy, but it is necessary to pay attention to the product that the increase of the other one or two markers may cause specific diseases, and the individual is reminded to improve this functional behavior. For example, if microalbumin is abnormally elevated, while the other three markers are at normal levels, it means that the individual is generally healthy, but intervention or improvement is needed to avoid the occurrence of diabetes, and attention should be paid to dietary habits, etc. The health status here is merely a relative concept.

### Cut-off setting

In some embodiments, the health cut-off value may vary depending on different countries, different living environments, and different ages, but the average health cut-off level can be tested or screened according to clinical testing, and then these markers above these health cut-off levels indicate overall unhealthy. Of course, when the concentration is very high, it may indicate that the higher the degree of being unhealthy, the better the risk of developing specific diseases. In some embodiments, the cut-off value of NPT in urine may be about 200 ng/mL, the cut-off value of HCY in urine may be 2000 ng/mL, the cut-off value of mALB in urine may be 40 ug/mL, and the cut-off value of DOP in urine may be 500 ng/mL. It can be understood that these cut-off values are merely estimated values and may be adjusted according to clinical requirements. Generally, the concentrations of markers of normal health levels for different people, different regions and different ages are within a range, and the cut-off value can be selected according to different purposes. In some embodiments, the corresponding cut-off value for each marker substance may be different. In some embodiments, the cut-off value of the urine adulteration test by NPT and the cut-off value of the overall health test may be the same or different, and may be adjusted according to different situations, and any adjusted value is also within the scope of protection of the present invention.

After the samples of the 4 different labeling substances are verified, a single marker can well distinguish overall healthy and overall unhealthy people. The AUC value of NPT is above 0.75, the AUC value of HCY is above 0.70, the AUC value of mALB is above 0.72, and the AUC value of DOP is above 0.75. The specific test method is to use a reagent strip method, and a specific cut-off is set in advance for testing.

The specific method of verification is collecting samples from the confirmed healthy sample population and non-healthy sample population (no other specific diseases, especially no cancer & infectious diseases, diabetes, mental diseases, and cardiovascular diseases among other specific diseases) in different stages, grouping and classifying the number of people developing specific diseases in the non-healthy people again, then performing testing to see if a better discrimination can be obtained, and then calculating the AUC values of the markers. The urine samples of these populations are all collected from existing stored samples collected continuously for many years in the sample center, these samples are all corresponding to specific individuals (no specific information of people, but there are numbers), and the samples stored at different times are tested and analyzed for the specificity, sensitivity and specific predictive performance of the 4 markers of the present invention. When the combined testing of the 4 markers is performed, it can better, with high sensitivity and high specificity, distinguish the overall health or unhealthy status of the population. With the combined testing of the 4 markers, the AUC can reach more than 0.8. Of course, the AUC value here is based on a limited number of samples for testing and settlement, but as the number of samples varies, the AUC value may change. However, through our experiments, it has been proved at least that the above 4 markers can distinguish between systemically healthy and unhealthy status, and intervention can be performed in advance to improve and avoid the occurrence of specific diseases.

In some embodiments, it is generally understood that physical health status includes physical or physiological health risks, as well as mental health risks, which interact and influence each other. At present, the well-known health status of the human body or the appearance of certain diseases, the psychological effect is an important factor. If you are under psychological pressure for a long time, it will easily lead to some specific diseases. On the contrary, if you have a positive attitude, you can improve your body's resistance and reduce the risk of diseases. The present invention finds that dopamine is directly associated with the development of cancer, infections, cardiovascular diseases, and diabetes. Therefore, in a preferred embodiment of the present invention, the 4 markers are simultaneously tested on individual fluid samples, so that the overall health of the individual can be evaluated more comprehensively. At present, there is no kit that can assess physical and psychological health status at the same time, and use urine samples for assessment at the same time. In the present invention, the combination of early prediction of physiological health risks and early psychological health risks can be used to carry out the testing, prediction and monitoring of systemic early health risks. When the sample is mainly urine, it is easily accessible; such self-testing can be performed at home, keeping an eye on one's own overall, early health status at any time.

In some embodiments, all the 4 markers are tested with lateral flow test strips, one test strip can be used to test the contents of the 4 markers in urine simultaneously, 2 test strips can be used, each test strip can test two markers, or four test strips can be used, each test strip can test a marker, the above different combinations or forms of test strips can be contacted with a liquid sample, such as urine, at the same time, and the same sample can be tested for a combination of multiple markers. In some embodiments, the test strip includes a sample application pad, a labeling pad, a testing pad, and a water absorbent pad, where the labeling pad contains antibodies capable of specifically binding to these markers, the antibodies are coupled to labels, and the analogs of these labeling substances are fixed on the testing pad, and when the sample contains the labeling substance, the analogous substances of the markers and the antibody-marker complexes compete on the testing pad for binding, thereby obtaining a test result by whether or not a color appears on the testing pad. If a color appears, it indicates a negative result, suggesting that it is lower than a preset cut-off value, and if no line appears, it indicates a positive result, suggesting that it is higher than a preset cut-off value.

### Antibody

The antibody for use in the present invention may be any antibody capable of specifically binding to an analyte in a sample, and in particular capable of specifically binding to the 4 marker substances of the present invention for testing the overall health of an individual: neopterin (NPT); homocysteine (HCY); dopamine (DOP); or microalbumin. The antibody that can be used as a binding agent can be any antibody known to those skilled in the art. The "antibody" can be an immunoglobulin molecule and an antigen binding part of the immunoglobulin molecule, that is, a molecule including an antigen-binding site that specifically binds to an analyte, an analyte analog or a ligand ("immune reaction"). This term also includes derivatives of antibodies in which the binding ability is retained, and also includes any protein containing a binding domain that is homologous or largely homologous to the binding domain of an immunoglobulin. These proteins may originate from natural substances, or they may be partially or fully synthesized. An antibody may be monoclonal or polyclonal. An antibody may be a member of any immunoglobulin type, including any human immunoglobulin type: IgG, IgM, IgA, IgD, IgG, and IgE. An "antibody fragment" is a derivative of an antibody or a portion of an antibody that is less than the full length. The antibody fragment can retain at least one significant site of the binding ability of the full-length antibody. Examples of the antibody fragment include an antigen-binding fragment (Fab), an Fab', an F(ab')₂, a single-chain variable fragment (scFv), a variable fragment (Fv), a disulfide-stabilized Fv (dsFv) dimer, and an Fd fragment, but are not limited to the above. The antibody fragment can be generated by any means. For example, the antibody fragment can be generated by enzymatic or chemical cleavage of a complete antibody, or can also be generated by recombination of genes encoding partial antibody sequences. In other words, the antibody fragment can be generated by partial or complete recombination. The antibody fragment can be any single-chain antibody fragment. In other words, the antibody fragment can include a plurality of peptide chains linked to each other, for example, by disulfide bonds. The antibody fragment can also be any one of multimolecular complexes. One functional antibody fragment typically includes at least about 50 amino acids, while more antibody fragments typically include at least about 200 amino acids. Single-chain Fvs (scFvs) are recombinant antibody fragments consisting only of a variable region of light chain (V_{L}) and a variable region of heavy chain (V_{H}) covalently bound to each other in a polypeptide chain. One of V_{L} and V_{H} has an amino terminal region. The length and composition of polypeptide chains are variable, and their length can bridge two variable domains to each other without significantly affecting the arrangement of atoms. Polypeptide chains are typically composed mainly of glycine and serine residue extensions, with some glutamic acid and lysine residues scattered to increase their solubility. The "dimer" refers to a bipolymer of single-stranded Fvs. The monomers of the dimer typically include peptide chains that are shorter than those of most single-chain Fvs, and they show a tendency to form the bipolymer.

The "Fv" fragment consists of one V_{H} and one V_{L} domain non-covalently linked to each other. The term "dsFv" here refers to an Fv including an intermolecular disulfide bond that stabilizes a V_{H}-V_{L} pair. The "F(ab')" fragment is a fragment of an antibody that is essentially identical to the fragment obtained by digesting an immunoglobulin (usually IgG) with pepsin at pH 4.0-4.5. This fragment can also be synthesized by recombination. The "Fab'" fragment is an antibody fragment that is essentially identical to the fragment obtained by reducing the disulfide bond linking the two heavy chains on the F(ab') fragment. The Fab' fragment can also be synthesized by recombination. The "Fab" fragment is an antibody fragment that is essentially identical to the fragment obtained by digesting an immunoglobulin (usually IgG) with papain. The Fab fragment can also be synthesized by recombination. The heavy chain fragment on the Fab fragment is the Fd fragment.

In the present invention, the antibody can specifically bind to an analyte in a liquid sample, such as urine, and the analyte here is the 4 labeling substances described above. In some embodiments, the antibody is also provided with a labeling substance, which is a substance indicating the number of antibodies, and which is a substance that can be read by means of an apparatus and equipment or in a manner visible to the naked eye. The labeling substance may be an example with a color, such as gold particles, latex particles, fluorescent substances, etc. These colored particles can be directly read and observed with the naked eye, or can be read through photography. Other labeling substances, such as fluorescent substances or otherwise, can be read with apparatuses and equipment. In some embodiments, an antibody of the present invention that specifically binds to the 4 markers bears a labeling substance, is treated and sprayed onto a labeling pad, and is present in a dry state such that the labeling pad, after being wet by the liquid sample, can dissolve in the liquid sample and flow downstream of the test strip as the liquid sample flows. When the sample contains any one of the above 4 marker substances, the antibody can bind to the marker substance to form a complex substance, for example, to form complexes of: labeling substance-anti-NPT antibody-NPT (in urine), and labeling substance-anti-HCY antibody-HCY; labeling substance-antibody against DOP-DOP; and labeling substance-anti-mALB antibody-mALB, where the antibody is a monoclonal antibody. These complexes flow to the testing area and compete for binding with the analogs of the analyte that are fixed on the testing area. Generally, the higher the concentration of the analyte in the sample, which exceeds a preset cut-off value, no lines appear in the testing area, and when the concentration is lower than a preset cut-off value, lines appear.

### Analogous substance of an analyte (marker substance)

The analogous substance of the analyte may be a substance similar to the analyte, but it may form a consortium with the antibody, and the antibody may form a consortium with the analyte. The analytes here are the 4 marker substances of the present invention. In some embodiments, the analog of the analyte exhibits competing ability with the analyte to bind to the antibody on the labeling area, or exhibits the same ability to bind to the antibody, or both differ in their ability to bind to the antibody. The binding ability between the analogous substance of the analyte and the antibody may be stronger or weaker than the binding force between the analyte and the antibody. The binding ability between the analogous substance of the analyte and the antibody may be greater or lower than the binding force between the analyte and the antibody. This binding ability can be selected or optimized according to the prior known techniques according to the needs of the test. For example, a binding molecule, such as a binding site or a member in a binding pair molecule, can be obtained by arbitrarily or non-arbitrarily mutating.

The analogous substance of the analyte may be a fragment of the analyte that retains some sites of the analyte. The labeling substance may be attached to these analogs by a linker including one binding site that is not present on the analyte, the analogous substance of the analyte and the labeling substance. For example, these sites may be recognized by some antibodies, but not by the analyte, the analogous substance of the analyte and the labeling substance. Optionally, these linkers can be recognized by antibodies and not by sites on other linkers. Such linkers include, but are not limited to, proteins, natural or synthetic polypeptides or some carbohydrates such as keyhole limpet hemocyanin (KLH), bovine gamma globulin (BGG), bovine serum albumin (BSA), bovine thyroglobulin (BTG), ovalbumin (OVA), sperm whale myoglobin (SWM), tetanus toxin (TT), methylated bovine serum albumin (mBSA), and rabbit serum albumin (RSA).

In some embodiments of the present invention, the analog of the marker substance may be an antigen, and the marker itself is a hapten, so that the marker is capable of causing an immune response. It is necessary to modify the marker to become an antigen, and the modification method is connecting some molecules through some chemical bonds to make it have immunogenicity, where these molecules may be any one of the above-described linkers. In some embodiments, the analogs of these markers of the present invention are fixed to the testing area and cannot move with the flow of the liquid. These analogous substances compete with the analyte for binding to the antibody bearing the labeling substance, so that on the testing area, if there is no colored line, the test result is positive, and if there is a colored line, the test result is negative. That is, a positive result indicates that the marker substance in the sample exceeds a predetermined cut-off value, and a negative result indicates that the labeling substance in the sample is lower than a preset cut-off value.

### Flow of a liquid

The flow of a liquid generally refers to the flow from one place to another. Under normal circumstances, the flow of a liquid in nature mostly depends on the action of gravity to flow from high places to low places. The flow here also depends on external forces, i.e., external gravity, which can become the flow under normal gravity. In addition to gravity, the flow of a liquid can also overcome gravity and make a movement from a low place to a high place. For example, a liquid can flow from a low place to a high place through extraction, compression or pressure received by it. Alternatively, a liquid against its own gravity in relation to pressure can flow.

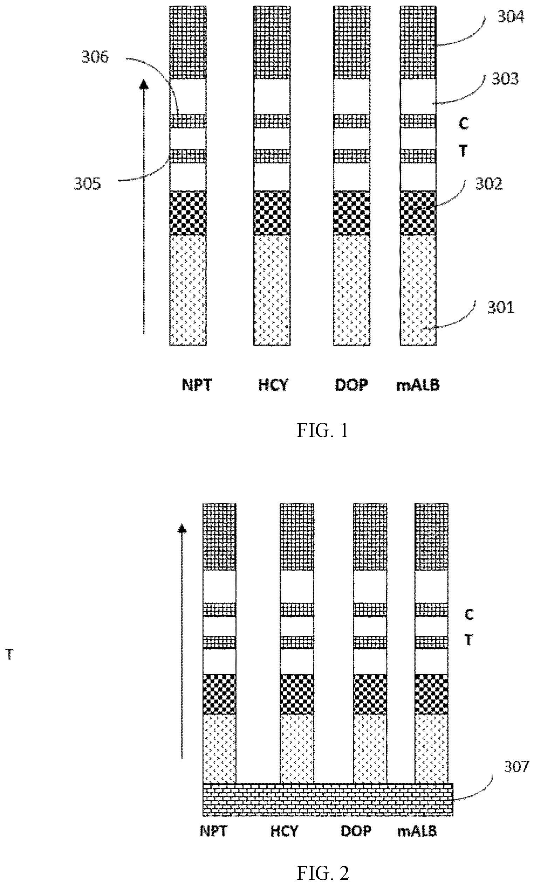

In some embodiments, four reagent strips as shown in FIG. 1 are provided, with each reagent strip detecting a marker. For example, one reagent strip is used to detect NPT, another test strip is used to detect HCY, and another test strip is used to detect DOP and mALB individually, separately. The specific method uses a competitive method similar to drug abuse for testing. If a line does not appear on the test line T, it indicates that these substances exceed a preset cut-off value, suggesting that the systemic early health status is not good. On the contrary, if a line appears on the test line T, it indicates that these substances are below a preset cut-off value, suggesting that the systemic health is good. During testing, a single test strip can be used for testing separately, or four test strips can be used to test the same sample at the same time.

In some embodiments, analogs of four analytes are fixed on the T-line, and the labeling pad is labeled with an antibody capable of specifically binding to the analyte in the sample. The labeling substance may be gold particles, latex particles or water-soluble dyes. The antibody on the labeling pad can follow the flow of the liquid. The principle of several other marker tests is the same as above. During testing, if any of NPT, HCY, DOP and mALB in the sample, such as a urine sample, exceeds a preset cut-off value, it means that the person tested has an overall and comprehensive health risk, but a specific disease type cannot be determined; and if all the 4 markers are positive, it means that the overall health risk is relatively high, and further professional detection is needed to determine if the person may have specific types of diseases.

FIG. 2 is an alternative method in which the test strips of the 4 markers are connected together by flow guide elements. When the sample is applied to the flow guide elements, the sample flows onto the 4 test strips respectively to complete the simultaneous test of the 4 markers in the fluid sample.

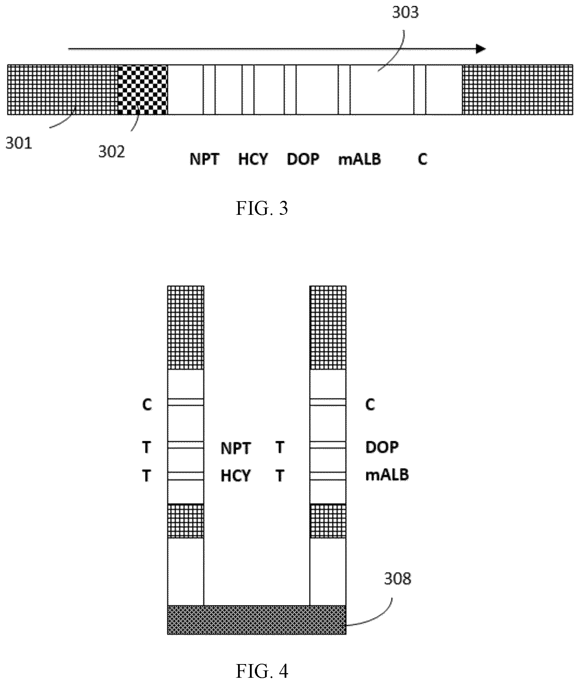

FIG. 3 is an alternative embodiment in which a test strip has 4 test lines, each test line corresponding to a marker substance of the present invention, and the labeling pad has a mixture of 4 different antibodies with color particles. During testing, urine is applied to the sample pad or the sample of the test strip is inserted into the urine to observe if the 4 test lines show a color or shade of color. If there is a color, a negative result indicates that the overall health of the individual is good, and if there is no color, it indicates that one or more markers are positive, indicating that the overall health is poor or not good.

FIG. 4 is another specific embodiment. Two marker substances are detected on two test strips respectively, and then connected together through flow guide pads. Two indicators can be tested at the same time on each test. Of course, 4 indicators can be tested at the same time.

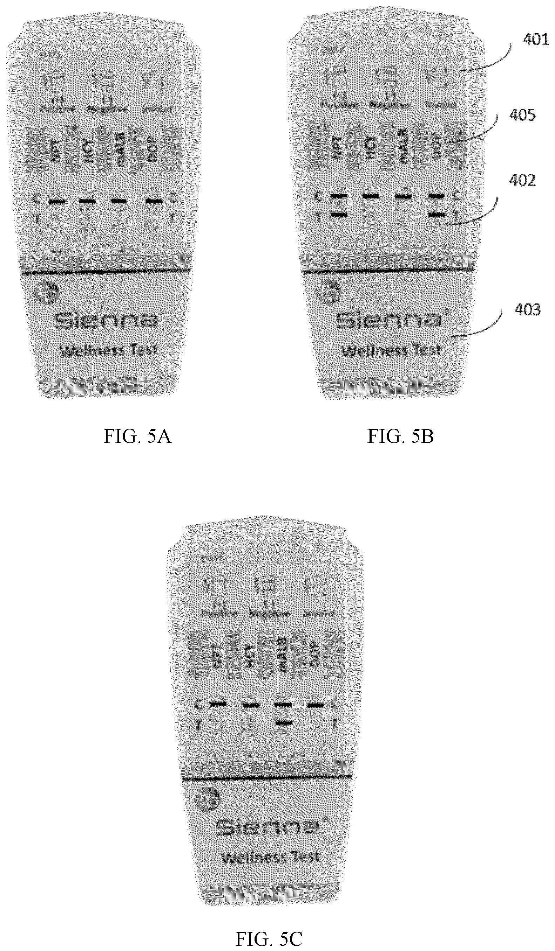

FIGS. 5-7 are a specific embodiment in which test strips are loaded into a test card and the sample extends from the test card, and each test strip is used to detect a marker substance. During testing, urine is collected with a urine cup, then the test card is inserted into the urine, and the test result is read after about 10 minutes. As long as one indicator is positive, the result is determined as positive (no T line appears), indicating that the systemic early health status is not very good. On the contrary, if the results are all negative (T lines appear), it means that the health status is very good. If the C line does not appear, the test result is invalid.

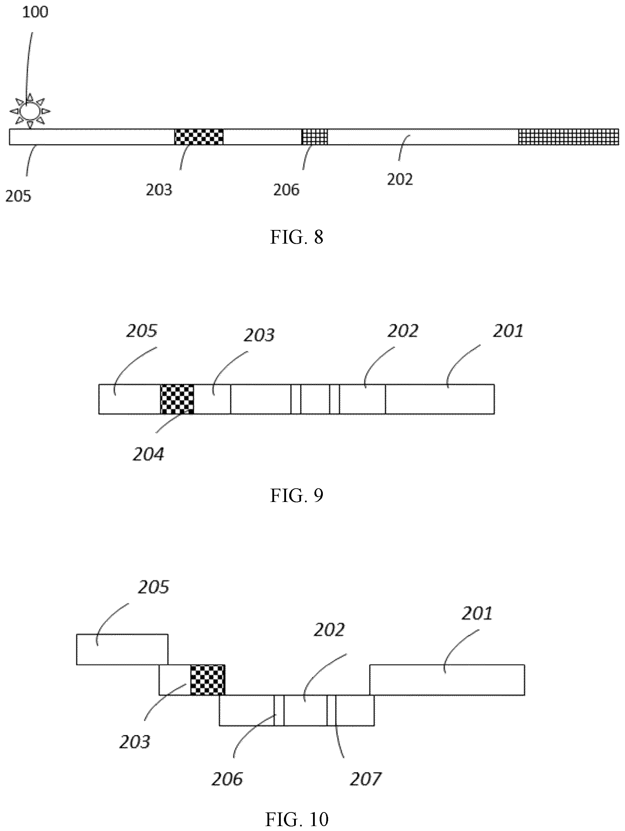

FIG. 8 is an explanatory diagram of the basic principle of the present invention, using NPT as an example to illustrate how to set up the test strip of the present invention. Of course, the other three indicators can also be detected using the same design principle as NPT or other similar principles.

### Example 1: Preparation of lateral flow detection device by immunoassay provided in the present invention

As shown in FIGS. 9 and 10, the lateral flow detection device prepared in this example for detecting the 4 markers by immunoassay includes a test strip, which is a sample area 101, a labeling area 102, a detection area 103, and a water absorption area 104 in sequence from upstream to downstream according to the direction of liquid flow, where the water absorption area is prepared by adopting general water absorbent filter paper as a water-absorbing pad; the sample area 101 uses a sample application pad, and the material of the sample application pad is glass fiber, so that the liquid sample is based on the glass fiber, and the liquid sample flows onto the labeling pad 102 by capillary force; the labeling area is formed into a labeling pad including an antibody coupled to labeling particles (e.g. gold particles, fluorescent particles, latex particles or dyes, or other colored labeling substances), and then the labeling mixture is sprayed onto a polyester film by a spraying device to form a labeling pad, and the labeling substance on the labeling pad is capable of flowing with the flow of the liquid; and the detection area adopts nitrocellulose membrane, dissolves the antigen of the detection line with buffer solution PBS, and then uses a membrane-spotting device to draw a line on the nitrocellulose membrane to make the distance between different antibodies 3-8 mm, then the nitrocellulose membrane is placed in an oven for drying later, and the antigen or other binding substances treated on the membrane are generally immobile, and the detection area includes a detection region 105 and a detection result control area 106.

After the preparation of the water absorption area 104, the sample area 101, the labeling area 102, and the detection area 103 are completed separately, the assembly is carried out, one end of the sample application pad is superimposed on one end of the labeling pad, the other end of the labeling pad is superimposed on the nitrocellulose membrane, and the nitrocellulose membrane at one end of the control line is superimposed by the water absorbent filter paper, so that the whole test strip is formed, and then assembled in the test card, where the sample pad extends from the test card for contacting the liquid sample, and the nitrocellulose membrane corresponds to the degree window for reading the test result.

The embodiments as below also belong to the current application:
1. Use of a biomarker for preparation of a reagent for predicting systemic, overall health of an individual, wherein the biomarker is selected from one or a combination of more than one of biomarkers of: neopterin; homocysteine; dopamine; or microalbuminuria.
2. The use according to clause 1, wherein the individual does not suffer from a specific disease.
3. The use according to clause 2, wherein the specific disease comprises one or more of cancer, an infectious disease, a cardiovascular disease, and diabetes.
4. The use according to clause 3, wherein the use comprises a reagent comprising an immune reagent, and the immune reagent comprises an antibody that specifically binds to a biomarker, and an analogous substance of the biomarker.
5. The use according to clause 4, wherein the antibody that specifically binds to the biomarker is further coupled with a labeling substance, and the labeling substance comprises a gold particle, a latex particle or a colored dye.
6. The use according to clause 5, wherein the antibody specifically binding to a biomarker is treated on a labeling pad and is capable of moving with flow of a liquid, and the analogous substance is fixed on a membrane and is incapable of moving with flow of a liquid.
7. The use according to clause 6, wherein the membrane is a nitrocellulose membrane.
8. A device for detecting an analyte in a liquid sample, comprising: a test element for detecting presence or a quantity of the analyte in the liquid sample, wherein the analyte is one or more of analytes of: neopterin; homocysteine; dopamine; or microalbuminuria.
9. The device according to clause 8, wherein a test strip is a lateral flow test strip.
10. The device according to clause 9, wherein the test strip is capable of simultaneously testing four analytes in the sample, and the analytes comprise a group of: neopterin; homocysteine; dopamine; or microalbuminuria.
11. The device according to clause 10, wherein the test strip is capable of simultaneously testing four analytes in the sample, and the analytes consist of analytes of: neopterin; homocysteine; dopamine; or microalbuminuria.
12. The device according to any one of clause s 8-11, wherein the test strip comprises a detection area and a labeling area, the labeling area comprises a first receptor capable of specifically binding to the analyte and capable of flowing with the liquid sample, and the detection area comprises a second receptor incapable of flowing with the liquid.
13. The device according to clause 12, wherein the second receptor competes with the analyte for binding to the first receptor.
14. The device according to clause 13, wherein the first receptor comprises an antibody, an antibody fragment, a polypeptide, or a polypeptide fragment, wherein the antibody is a monoclonal antibody.
15. The device according to clause 13, wherein the second receptor is an analog of the analyte or an antigenic substance of the analyte.
16. The device according to clause 15, wherein the analog of the analyte comprises a linker molecule, and the molecule is selected from one of molecules of: keyhole limpet hemocyanin (KLH), bovine gamma globulin (BGG), bovine serum albumin (BSA), bovine thyroglobulin (BTG), hen egg lysozyme (HEL), ovalbumin (OVA), sperm whale myoglobin (SWM), tetanus toxin (TT), methylated bovine serum albumin (mBSA), and rabbit serum albumin (RSA).
17. The device according to clause 14, wherein the first receptor further comprises a labeling substance, and the labeling substance comprises a gold particle, a latex particle, a water-soluble dye, or a fluorescent substance.
18. The device according to clause 8, wherein the liquid sample is a saliva, urine or blood sample.
19. The device according to clause 8, comprising: 4 test elements, with each test strip test element for testing presence or a quantity of an analyte in the liquid sample, wherein the analyte comprises 4 analytes of: neopterin; homocysteine; dopamine; or microalbuminuria.
20. The device according to clause 8, wherein the device comprises one test strip, and the test strip is capable of simultaneously testing 4 analytes in the liquid sample: neopterin; homocysteine; dopamine; or microalbuminuria.
21. The device according to v 8, wherein the device comprises 2 test strips, each test strip tests 2 different analytes, and the analytes are selected from a combination of 2 analytes of: neopterin; homocysteine; dopamine; or microalbuminuria.
22. The device according to clause 8 wherein the test strip is capable of simultaneously testing four analytes in the sample, and the analytes comprise a group of: neopterin; homocysteine; dopamine; or microalbuminuria.
23. The device according to clause 8, wherein the test strip is capable of simultaneously testing four analytes in the sample, and the analytes consist of analytes of: neopterin; homocysteine; dopamine; or microalbuminuria.

All patents and publications mentioned in the specification of the present invention indicate that these are disclosed techniques in the art and can be used by the present invention. All patents and publications cited herein are likewise listed in the references as if each publication is specifically and separately referenced. The present invention described herein may be implemented in the absence of any one or more elements, and one or more limitations, which are not specifically stated herein. For example, the terms "comprising", "consisting essentially of" and "consisting of" in each of the examples herein may be replaced by the remaining two terms of one of the two. The term "one" herein only means "a", and does not exclude the inclusion of only one, and may mean the inclusion of two or more. The terms and expressions employed herein are descriptive and are not limited thereto, and there is no intention herein to indicate that the terms and interpretations described herein exclude any equivalent features, but it can be noted that any appropriate changes or modifications can be made within the scope of the present invention and claims. It can be understood that the embodiments described in the present invention are preferred embodiments and features, and any person skilled in the art can make some modifications and changes based on the essence of the description of the present invention. These modifications and changes are also considered to be within the scope of the present invention and the scope limited by the independent claims and the dependent claims.

## Claims

1. A method for predicting or determining overall, comprehensive or systemic health of an individual, comprising:
providing a liquid sample of the individual;
testing a quantity or a concentration of an analyte in the liquid sample; and
determining the overall health of the individual according to the concentration or quantity tested,
wherein the analyte comprises a combination of one or more of markers of: neopterin;
homocysteine; dopamine; or microalbuminuria.

2. The method according to claim 1, wherein the method further comprises comparing the concentration and the quantity of the analyte tested with a preset cut-off value, and predicting the overall, comprehensive or systemic health of the individual according to a result of the comparison.

3. The method according to claim 2, wherein when a concentration of any one analyte exceeds the preset cut-off value, the individual is indicated to be overall unhealthy; or when a concentration of any analyte is lower than the preset cut-off value, the individual is indicated to be overall healthy.

4. The method according to any one of claims 1-3, wherein when concentrations of the 4 markers all exceed the preset cut-off value, the individual is indicated to be overall, systemically and comprehensively physically very unhealthy, and the individual is very likely to be at risk of suffering from a specific disease in the future.

5. The method according to claim 4, wherein the specific disease comprises one or more of cancer, an infectious disease, a cardiovascular disease, and diabetes.

6. The method according to any one of claims 1-5, wherein when a concentration of the neopterin (NPT) exceeds the preset cut-off value, the individual is indicated to be overall unhealthy, and the individual is likely to be at risk of suffering from cancer or an infectious disease in the future.

7. The method according to any one of claims 1-6, wherein when a concentration of the homocysteine exceeds the preset cut-off value, the individual is indicated to be overall unhealthy, and the individual is likely to suffer from a cardiovascular disease in the future.

8. The method according to any one of claims 1-7, wherein when a concentration of the microalbuminuria exceeds the preset cut-off value, the individual is indicated to be overall unhealthy, and the individual is likely to suffer from diabetes in the future.

9. The method according to any one of claims 1-8, further comprising providing a test device, wherein the device comprises 4 lateral flow test strips, and each test strip is capable of testing one of the 4 marker substances, allowing the 4 test strips to simultaneously contact the liquid sample, and then obtaining a test result.

10. The method according to claim 9, wherein if a test result of any one test strip is negative, the concentration of the analyte tested is indicated to be lower than the preset cut-off value, and the individual is indicated to be overall healthy; or if a test result of a test area of any one test strip is positive, the concentration of the analyte tested is indicated to be higher than the preset cut-off value, and the individual is indicated to be overall unhealthy.

11. The method according to any one of claims 9-10, wherein if a test result of each test strip is negative, concentrations of the 4 analytes tested are indicated to be lower than the preset cut-off value, and the individual is indicated to be overall very healthy; or if a test result of a test area of each test strip is positive, concentrations of the analytes tested are indicated to be higher than the preset cut-off value, and the individual is indicated to be overall very unhealthy.

12. The method according to any one of claims 1-11, wherein the liquid sample comprises one of a urine, saliva or blood sample.

13. The method according to any one of claims 9-12, wherein if a test result of each test strip is negative, concentrations of the 4 analytes tested are indicated the individual is at no risk of suffering from a specific disease or at little risk of suffering from a specific disease.

14. The method according to claim 13, wherein the specific disease comprises one or more of cancer, an infectious disease, a cardiovascular disease, and diabetes.

15. A method for testing whether urine is adulterated, comprising: testing a quantity of neopterin (NPT) in the urine, and determining whether the urine is adulterated according to the quantity or a concentration of the neopterin.

16. The method according to claim 15, wherein when the NPT tested is lower than a preset cut-off or is 0, the urine is indicated to be adulterated.

17. The method according to claim 16, wherein when the NPT tested is higher than a preset cut-off, an individual is indicated to be overall unhealthy and at risk of suffering from a specific disease, or the urine is indicated to be unadulterated.

18. The method according to any one of claims 15-17, wherein using a test strip to test whether the urine is adulterated, wherein the test strip has a labeling area and a test area, an antibody that specifically binds to the NPT is treated on the labeling area, and the test area is fixed with an analogous substance of the NPT.

19. The method according to claim 18, wherein when a positive rest result in the test area on the test strip, the NPT is indicated to be higher than a preset cut-off value, and the urine is indicated to be unadulterated.

20. The method according to claim 18, wherein when a negative test result in the test area on the test strip, the NPT is indicated to be lower than a preset cut-off value, and the urine is indicated to be adulterated.
